# EUROPEAN PATENT APPLICATION

(11) **EP 4 401 015 A1**
(43) Date of publication of application: **17.07.2024**
(21) Application number: 22867344.8
(22) Date of filing: 06.09.2022
(51) Int. Cl.: G06N 20/00, G01N 27/26, G01N 33/18

(54) **MEASUREMENT SYSTEM, MEASUREMENT SYSTEM ANOMALY DETERMINATION METHOD, AND MEASUREMENT SYSTEM ANOMALY DETERMINATION PROGRAM**

(30) Priority: 09.09.2021 JP 2021146925; 30.03.2022 JP 2022054985
(71) Applicant: Horiba Advanced Techno, Co., Ltd., Kyoto-shi, Kyoto 6018551 (JP)
(72) Inventor: TANAKA, hideaki, Kyoto-shi, Kyoto 601-8551 (JP); KANDA, hiroshi, Kyoto-shi, Kyoto 601-8551 (JP)
(74) Representative: Isarpatent
(86) International application number: PCT/JP2022/033404
(87) International publication number: WO 2023/038022

(57) **Abstract**

A measurement system includes a measurement instrument 20 including at least a first sensor 21 and a second sensor 22, a prediction value estimation unit 35 that estimates a prediction value of the second sensor 22 based on an actual measured value of the first sensor 21 using a machine learning model that has learned a relationship between the actual measured value of the first sensor 21 and an actual measured value of the second sensor 22, and an anomaly determination unit 36 that compares the prediction value of the second sensor 22 and the actual measured value of the second sensor 22 to determine which of the machine learning model or the measurement instrument 20 has an anomaly.

## Description

### Technical Field

The present invention relates to a measurement system that measures properties of a sample such as water quality, a measurement system anomaly determination method, and a measurement system anomaly determination program.

### Background Art

For example, in a liquid analysis device as in Patent Literature 1 equipped with a sensor that measures water quality, conventionally, if an obvious anomaly such as off-scaling of an output value of the sensor occurs, a user can also determine that maintenance is necessary for the sensor.

However, even in a case where an obvious anomaly does not occur in the sensor, an output value of the sensor may deviate from a value to be originally output due to contamination of the sensor, a malfunction of a component affecting the output value of the sensor, or the like.

In such a case, it is difficult for the user to determine whether an output value of the sensor has changed due to dirt, a defect, or the like of the sensor or another component.

### Citation List

### Patent Literature

Patent Literature 1: JP 2015-25794 A

### Summary of Invention

### Technical Problem

Further, in recent years, it has also been considered to automatically determine maintenance of a measurement system utilizing machine learning. In this machine learning, supervised learning is performed by using a combination of past sensor output values and measured values obtained from the output values as training data (learning data set). A method for comparing a prediction value obtained by the machine learning with an actual measured value and determining the necessity of maintenance can be considered.

However, in a case where unknown data that greatly deviates from the learning data set due to a change in the tendency of the sample or the like is input, the prediction value estimated in a machine learning model may be greatly different from the actual measured value. As a result, even in a case where the measurement system requires no maintenance, a determination is made that the measurement system requires maintenance.

Therefore, the present invention has been made to solve the above problem, and it is a main object of the present invention to determine the necessity of maintenance of a measurement instrument or the necessity of relearning of a machine learning model in a measurement system utilizing machine learning.

### Solution to Problem

That is, a measurement system according to the present invention includes a measurement instrument including at least a first sensor and a second sensor, a prediction value estimation unit that estimates a prediction value of the second sensor based on an actual measured value of the first sensor using a machine learning model that has learned a relationship between the actual measured value of the first sensor and an actual measured value of the second sensor, and an anomaly determination unit that compares the prediction value of the second sensor with the actual measured value of the second sensor to determine which of the machine learning model or the measurement instrument has an anomaly.

According to the measurement system configured as described above, the prediction value of the second sensor is estimated based on the actual measured value of the first sensor using the machine learning model, the estimated prediction value of the second sensor is compared with the actual measured value of the second sensor, and the determination is made which of the machine learning model or the measurement instrument has an anomaly. Therefore, the necessity of maintenance of the measurement instrument can be accurately determined. Further, in a case where the determination is made that the machine learning model has an anomaly, a detection can be made that the tendency of the sample has changed after a time of creating the machine learning model, and the machine learning model can also perform relearning.

As a specific aspect of the anomaly determination, the anomaly determination unit desirably determines whether a difference or a ratio between the prediction value of the second sensor and the actual measured value of the second sensor falls within a predetermined tolerance range. In a case where the difference or the ratio is out of the tolerance range, a determination is desirably made which of the machine learning model or the measurement instrument has an anomaly.

Specifically, the anomaly determination unit desirably determines which of the machine learning model or the measurement instrument has an anomaly using a non-parametric density estimation method.

As a specific aspect of the anomaly determination using the non-parametric density estimation method, the anomaly determination unit desirably determines that the machine learning model has an anomaly in a case where determining that an anomaly occurs in a region where a data density of a learning data set with which the machine learning model is created is equal to or greater than a predetermined threshold, and desirably determines that that the measurement instrument or the machine learning model has an anomaly in a case where determining that an anomaly occurs in a region where the data density is less than the threshold. Note that, in the case where the determination is made that the measurement instrument or the machine learning model has an anomaly in the region where the data density is less than the threshold, a determination can be made that the machine learning model is likely to have an anomaly when no anomaly is confirmed by analyzing internal data of the measurement instrument.

In addition, the measurement system of the present invention desirably further includes a machine learning unit that creates the machine learning model using a learning data set including the actual measured value of the first sensor and the actual measured value of the second sensor.

Since the measurement system includes the machine learning unit as described above, the machine learning model can be created by using the learning data set obtained by a sample measured by a user.

The machine learning unit desirably causes the machine learning model to perform relearning in the case where the anomaly determination unit determines that the machine learning model has an anomaly.

By causing the machine learning model to perform relearning as described above, the reliability of the machine learning model can be improved. By estimating the prediction value of the second sensor using the machine learning model that has performed relearning, the necessity of maintenance of the measurement instrument can be determined more accurately.

In order to prompt the user to perform maintenance, a maintenance signal for prompting the user to perform maintenance of the measurement instrument is desirably output in the case where the anomaly determination unit determines that the measurement instrument has an anomaly.

It is conceivable that the first sensor and the second sensor each measure any one of conductivity, specific resistance, oxidation-reduction potential (ORP), chemical oxygen demand (COD), turbidity, chromaticity, temperature, pressure, or oil film of a sample, or concentration of a predetermined component contained in the sample.

Here, the user performs maintenance of the sensors, which is a series of operations including cleaning of the sensors, calibration of the sensors, replacement of the sensors, relearning of the machine learning model, and a change of a maintenance period, with regular period. Conventionally, during the maintenance of the sensors, a degradation tendency which is a temporal change of degradation of each of the sensors is understood, and a maintenance period that is reflective of the degradation tendency is calculated for each of the sensors alone. On the other hand, in the method for calculating the maintenance period that is reflective of the degradation tendency for each of the sensors alone, the degradation tendency of each of the plurality of sensors has to be understood. Meanwhile, in the same measurement system, degradation tendencies of the plurality of sensors belonging to the same measurement system may be similar to each other. Here, the same measurement system refers to, for example, a flow path without inflow from the upstream to the downstream in the same tank or water treatment facility. In this case, when the user understands the degradation tendency of a certain sensor, it is conceivable that the user estimates the degradation tendencies of other sensors based on the degradation tendency of the sensor.

In order to cope with the above description, the measurement system further includes a degradation determination unit that determines, based on reference degradation data indicating a relationship between a state prediction value obtained by predicting a state of the first sensor and time and actual measured degradation data indicating a relationship between a state actual measured value obtained by actually measuring a state of the first sensor and time, whether a difference between the state actual measured value of the first sensor and the state prediction value falls within a tolerance range, the degradation determination unit outputting an out-of-tolerance range degradation signal indicating that the state of the first sensor is out-of-tolerance range degradation in a case where the degradation determination unit determines that the difference is out of the tolerance range.

With such a configuration, since the user recognizes the out-of-tolerance range degradation signal indicating the out-of-tolerance range degradation of the first sensor output by the measurement system, the user can estimate that the out-of-tolerance range degradation occurs also in the other sensors in the measurement system. As a result, the user can check degradation states of the other sensors in the same measurement system even in a maintenance period that has not been calculated in advance.

It is also conceivable that the measurement system further includes an actual measured degradation data calculation unit that calculates first actual measurement degradation data indicating a relationship between the state actual measured value of the first sensor and time and second actual measured degradation data indicating a relationship between the state actual measured value of the second sensor and time, and a maintenance period calculation unit that calculates a maintenance period of the first sensor and a maintenance period of the second sensor based on the first actual measured degradation data and the second actual measured degradation data.

In such a case, the measurement system calculates the maintenance periods of the first sensor and the second sensor based on the first actual measured degradation data and the second actual measured degradation data, and thus the maintenance periods of the first sensor and the second sensor can be changed to ones adapted to the state actual measured values.

As a specific aspect of the present invention, the measurement system can be used for water quality analysis.

Further, a measurement system anomaly determination method according to the present invention for measuring a sample using at least a first sensor and a second sensor includes estimating a prediction value of the second sensor based on an actual measured value of the first sensor using a machine learning model that has learned a relationship between the actual measured value of the first sensor and an actual measured value of the second sensor, and comparing the prediction value of the second sensor with the actual measured value of the second sensor to determine which of the machine learning model or the measurement instrument has an anomaly.

Further, a measurement system anomaly determination program according to the present invention, the measurement system having at least a first sensor and a second sensor, causes a computer to function as a prediction value estimation unit that estimates a prediction value of the second sensor based on an actual measured value of the first sensor using a machine learning model that has learned a relationship between the actual measured value of the first sensor and an actual measured value of the second sensor, and as an anomaly determination unit that compares the prediction value of the second sensor with the actual measured value of the second sensor to determine which of the machine learning model or the measurement instrument has an anomaly. Advantageous Effects of Invention

According to the present invention, the necessity of maintenance of a measurement instrument or the necessity of relearning of a machine learning model can be determined in a measurement system utilizing machine learning.

### Brief Description of Drawings

FIG. 1 is a schematic view illustrating a configuration of a measurement device according to a first embodiment of the present invention.
FIG. 2 is a diagram illustrating a physical configuration of an information processing apparatus according to the first embodiment.
FIG. 3 is a diagram illustrating a functional block of the information processing apparatus according to the first embodiment.
FIG. 4 is a flowchart of an anomaly determination method according to the first embodiment.
FIG. 5(a) is a graph illustrating learning data sets of a sensor A and a sensor B, and FIG. 5(b) is a diagram illustrating kernel density according to the first embodiment.
FIG. 6 is an analysis graph of the sensor A and the sensor B according to the first embodiment.
FIG. 7(a) is an analysis graph of the sensor A and the sensor B, FIG. 7(b) is an analysis graph of the sensor A and a sensor C, and FIG. 7(c) is an analysis graph of the sensor B and the sensor C.
FIG. 8 is a diagram illustrating a functional block of an information processing apparatus according to a second embodiment.
FIG. 9 is a flowchart of an anomaly determination method according to the second embodiment.
FIG. 10 is a graph of state prediction values and state actual measured values with respect to sensitivities of the first sensor and the second sensor according to the second embodiment.
FIG. 11 is a correspondence table of the state prediction values and the state actual measured values with respect to the sensitivities of the first sensor and the second sensor according to the second embodiment.

### Description of Embodiments

### <First embodiment>

Hereinafter, a first embodiment which is an embodiment of a measurement system according to the present invention will be described with reference to the drawings.

### <Basic configuration of measurement system>

A measurement system 100 according to the present embodiment measures one or a plurality of measurement items of a liquid sample (sample) such as clean water or sewage in, for example, a water treatment facility.

Here, examples of the measurement items include indexes related to water quality, such as a concentration of a predetermined component, such as hydrogen ion (H⁺), ammonium nitrogen (NH₄-N), residual chlorine, dissolved oxygen (DO), salt, fluoride ion, nitrate ion (NO₃⁻), hydrogen fluoride (HF), potassium hydroxide (KOH), tetramethylammonium hydroxide (TMAH), dissolved ozone, phosphoric acid (H₃PO₄), citric acid, ammonia (NH₃), nitrogen (N₂), phosphorus (P), or silica (SiO₂), a conductivity, a specific resistance, an oxidation-reduction potential (ORP), a chemical oxygen demand (COD), turbidity, chromaticity, temperature, pressure, or an oil film of the sample.

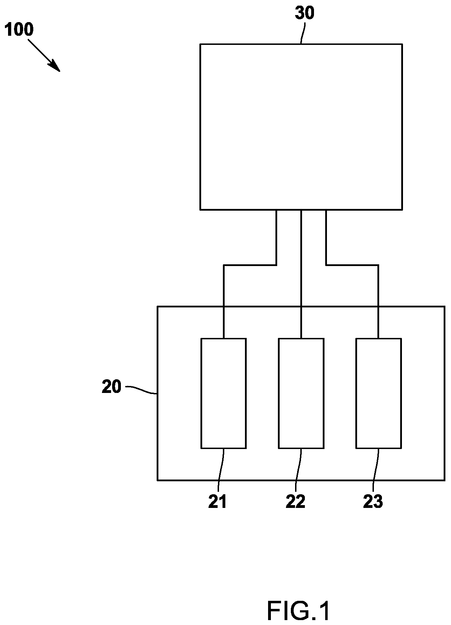

Specifically, as illustrated in FIG. 1, the measurement system 100 includes a measurement instrument 20 including at least a first sensor and a second sensor, and an information processing apparatus 30 that processes information output from the measurement instrument 20.

### <Configuration of measurement instrument 20>

The measurement instrument 20 measures one or a plurality of measurement items, and includes a plurality of sensors 21 to 23 respectively corresponding to the measurement items. In FIG. 1, the measurement instrument 20 has a configuration including the three sensors 21 to 23 different from each other, but may have a configuration including two sensors different from each other, or may have a configuration including four or more sensors different from each other. In addition, the measurement instrument 20 may include a plurality of the same sensors that are provided at different measurement places. Hereinafter, the three sensors 21 to 23 are also referred to as a first sensor 21, a second sensor 22, and a third sensor 23.

Each of the sensors 21 to 23 of the measurement instrument 20 can be appropriately changed in accordance with a measurement purpose. Examples of the sensors 21 to 23 of the measurement instrument 20 include an oxidation-reduction potential sensor, a potential of hydrogen (pH) sensor, a DO sensor, a sludge turbidity sensor (MLSS measurement device), an ammonia concentration sensor, and a temperature sensor. Further, the plurality of sensors 21 to 23 of the measurement instrument 20 may be a single measurement device unitized by being held in a single casing, or may be separate measurement devices provided independently from each other. Note that in the case of the separate measurement devices, for example, the measurement devices may be disposed at different locations such as different aeration tanks of water treatment equipment or different locations of sample piping.

Output values output respectively from sensors 21 to 23 of the measurement instrument 20 configured as described above are processed by the information processing apparatus 30. Note that the output values respectively from the sensors 21 to 23 can be transmitted to the information processing apparatus 30 in a wired or wireless manner.

### <Configuration of information processing apparatus 30>

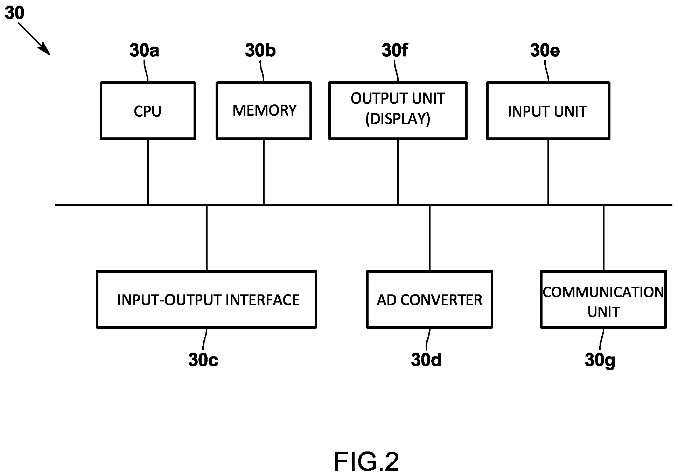

As illustrated in FIG. 2, the information processing apparatus 30 is a computer including a central processing unit (CPU) 30a, a memory 30b, an input-output interface 30c, an analog-to-digital (AD) converter 30d, an input unit 30e such as a keyboard, a display unit 30f such as a display, a communication unit 30g that performs communication via a network, and the like.

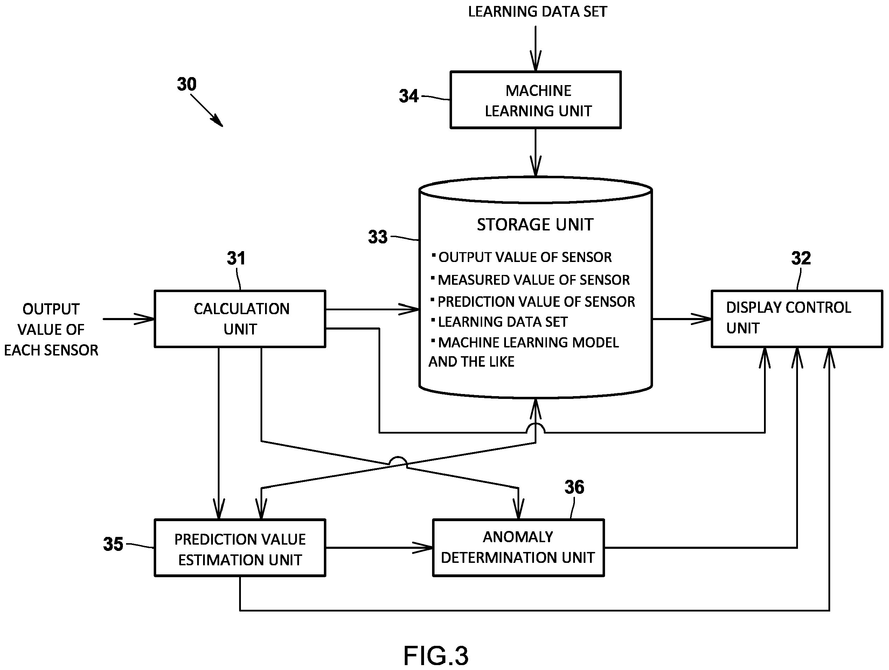

The information processing apparatus 30 delivers functionalities as a calculation unit 31, a display control unit 32, and a storage unit 33 as illustrated in FIG. 3 with cooperation of the CPU 30a and peripheral devices based on a program stored in a predetermined area of the memory.

The calculation unit 31 calculates the measured values of the measurement items based on the output values respectively from the sensors 21 to 23 of the measurement instrument 20. The measured values of the sensors 21 to 23 calculated by the calculation unit 31 are transmitted to the display control unit 32 and the storage unit 33.

The display control unit 32 displays the output values or measured values of the sensors 21 to 23 of the measurement instrument 20 on the display 30f. Specifically, for example, the display control unit 32 can display a graph having one axis (horizontal axis) indicating time and the other axis (vertical axis) indicating the output values or measured values on the display 30f, and can display a temporal change of the output values or measured values respectively from the sensors 21 to 23 on the graph. Further, the display control unit 32 can also display a list of measured values such as instantaneous values of the sensors 21 to 23 of the measurement instrument 20.

The storage unit 33 stores the output values, measured values, and the like of the sensors 21 to 23 of the measurement instrument 20. The various data stored in the storage unit 33 can be displayed on the display 30f by the display control unit 32.

### <Anomaly determination function using machine learning >

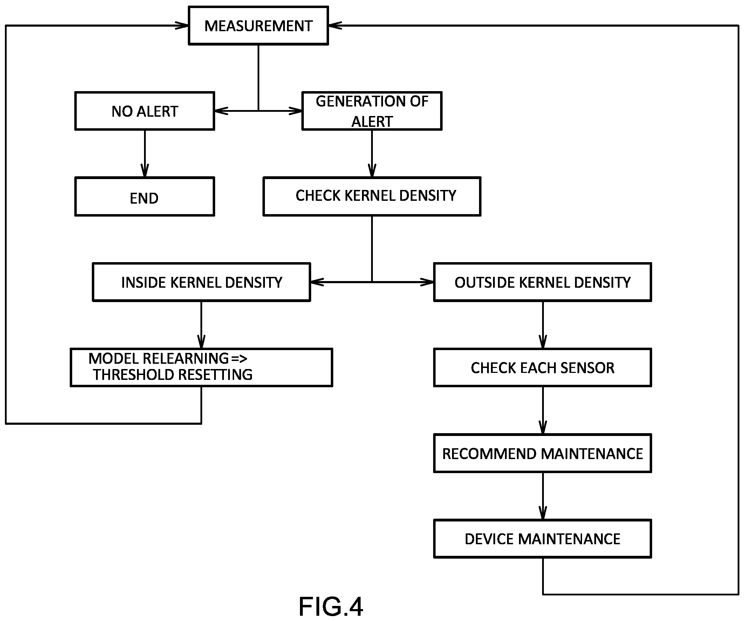

Furthermore, as illustrated in FIG. 3, the information processing apparatus 30 delivers functionalities as a machine learning unit 34, a prediction value estimation unit 35, and an anomaly determination unit 36. Hereinafter, the functions of the machine learning unit 34, the prediction value estimation unit 35, and the anomaly determination unit 36 will be described with reference to the flowchart of an anomaly determination method illustrated in FIG. 4. Further, for convenience of description, two of the three sensors 21 to 23, namely, the first sensor 21 and the second sensor 22, will be described, but the same applies to two sensors including the second sensor 22 and the third sensor 23, and two sensors including the first sensor 21 and the third sensor 23.

The machine learning unit 34 creates a machine learning model that has learned the relationship between the actual measured value of the first sensor 21 and the actual measured value of the second sensor 22 using a learning data set including a past actual measured value of the first sensor 21 and a past actual measured value of the second sensor 22.

Note that, as a learning algorithm of the machine learning unit 34, it is conceivable to use a support vector machine, a self-organizing map, an artificial neural network, a decision tree, a random forest, a k-means method, a k-nearest neighbor method, a genetic algorithm, a Bayesian network, or a deep learning method.

Here, the past measured value of the first sensor 21 is a past output value or measured value of the first sensor 21, and the past measured value of the second sensor 22 is a past output value or measured value of the second sensor 22. Note that the learning data set may be a combination of the past output value of the first sensor 21 and the past output value of the second sensor 22, a combination of the past measured value of the first sensor 21 and the past measured value of the second sensor 22, or a combination of the past output value of one sensor and the past measured value of the other sensor.

The prediction value estimation unit 35 estimates the prediction value of the second sensor 22 based on the actual measured value of the first sensor 21 using the machine learning model created by the machine learning unit 34. Note that the prediction value of the second sensor 22 is an output value or a measured value of the second sensor 22 predicted to be obtained at the same time as the time at which the measured value of the first sensor 21 is measured.

The anomaly determination unit 36 compares the prediction value of the second sensor 22 estimated by the prediction value estimation unit 35 with an actual measured value obtained by actually measuring the sample using the second sensor 22, and determines which of the machine learning model or the measurement instrument 20 has an anomaly.

Specifically, the anomaly determination unit 36 determines whether a difference or a ratio between the prediction value of the second sensor 22 and the actual measured value of the second sensor 22 falls within a predetermined tolerance range, and determines which of the machine learning model or the measurement instrument 20 has an anomaly in a case where the difference or the ratio is outside the tolerance range ("alert generation" in FIG. 4). Note that the tolerance range can be set in advance as, for example, a range of ± 10% of the prediction value.

Here, the anomaly determination unit 36 determines which of the machine learning model or the measurement instrument 20 has an anomaly using a non-parametric density estimation method. As the non-parametric density estimation method, for example, use of a kernel density estimation method is considered.

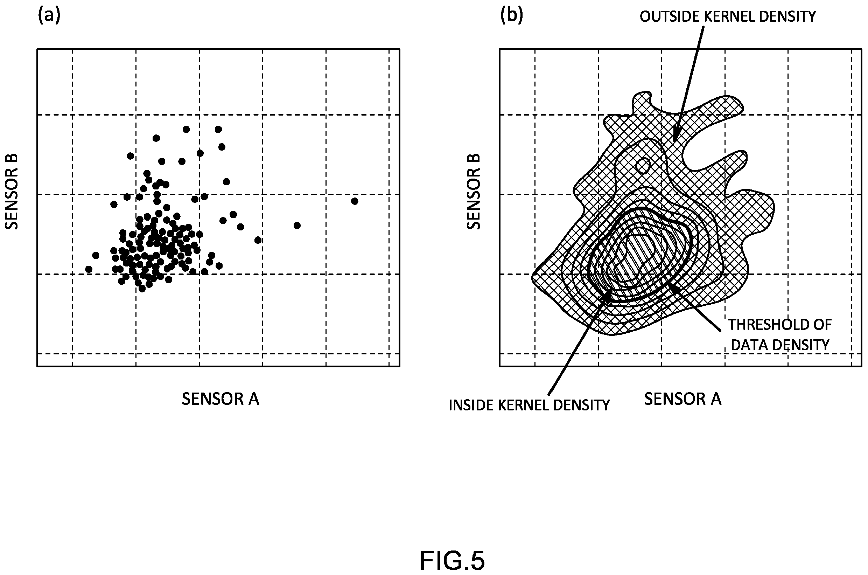

More specifically, as illustrated in FIG. 5(b), the anomaly determination unit 36 determines that the machine learning model has an anomaly in a case where determining an anomaly occurs in a region (inside the kernel density) where the data density of the learning data set with which the machine learning model is created is equal to or greater than a predetermined threshold (for example, 0.3) ("inside the kernel density" in FIG. 4).

Then, in a case where the determination is made that the machine learning model has an anomaly, the anomaly determination unit 36 outputs a relearning signal for prompting the user to make the machine learning model to perform relearning. Here, the relearning signal is displayed on the display, for example. When the user inputs a relearning command, the machine learning unit 34 makes the machine learning model perform relearning by using the updated learning data set (newly accumulated learning data set) and sets a threshold for determining an anomaly in the anomaly determination unit 36 ("model relearning □ threshold resetting" in FIG. 4). Note that each threshold of the anomaly determination unit 36 may be optionally set by the user.

On the other hand, as illustrated in FIG. 5(b), in a case where the determination is made that an anomaly occurs in the region where the data density is less than the threshold (outside the kernel density) ("outside the kernel density" in FIG. 4), the anomaly determination unit 36 determines that the machine learning model or the measurement instrument 20 has an anomaly. The anomaly determination unit 36 then checks whether the first sensor 21 or the second sensor 22 has an anomaly ("check of each sensor" in FIG. 4). Note that if no anomaly is checked in the first sensor 21 or the second sensor 22, the determination is made that the machine learning model has an anomaly, and as described above, the relearning signal for prompting the user to make the machine learning model perform relearning is output.

Here, in a case where the determination is made that the first sensor 21 or the second sensor 22 has an anomaly, the anomaly determination unit 36 outputs the maintenance signal for prompting the user to perform maintenance of the measurement instrument 20 ("recommendation of maintenance" in FIG. 4). For example, the anomaly determination unit 36 integrates the data density with the difference between the prediction value and the actual measured value, compares the integrated value (data density × (prediction value - actual measured value)) with a predetermined maintenance threshold, and outputs a maintenance signal. Here, the maintenance signal, which is displayed on the display, for example, may be a signal indicating that the measurement instrument 20 has an anomaly or a signal indicating a maintenance time. As a result, the user performs maintenance on the measurement instrument 20 determined to have an anomaly ("apparatus maintenance" in FIG. 4).

Here, in a case where an anomaly is detected by the anomaly determination unit 36, the following processing can also be performed.

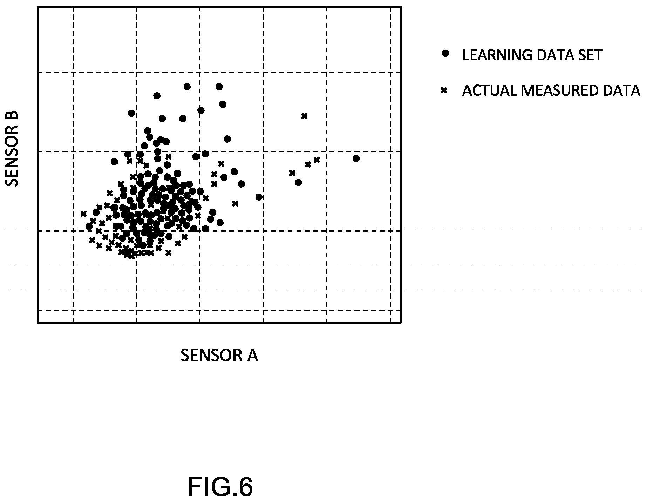

For example, as illustrated in FIG. 6, time-series data including the output values or actual measured values of the two sensors (the sensor A and the sensor B) selected from the plurality of sensors 21 to 23 can be plotted on a graph (hereinafter, analysis graph) having two sensor axes (a horizontal axis represents the sensor A, and a vertical axis represents the sensor B), and data (learning data) of the two sensors (the sensor A and the sensor B) included in the learning data set can be plotted on the analysis graph.

As described above, the cause of the deviation of the prediction result can be surmised by plotting the actual output values or measured values (actual measured data) of the two sensors (the sensor A and sensor B) and the data (learning data) of the two sensors (the sensor A and sensor B) on the analysis graph. For example, in a case where the actual measured data is plotted in a region having a small amount of learning data, it can be surmised that the tendency of the sample has changed. In addition, in a case where only actual measured data of a specific sensor changes, an anomaly of the specific sensor can be surmised.

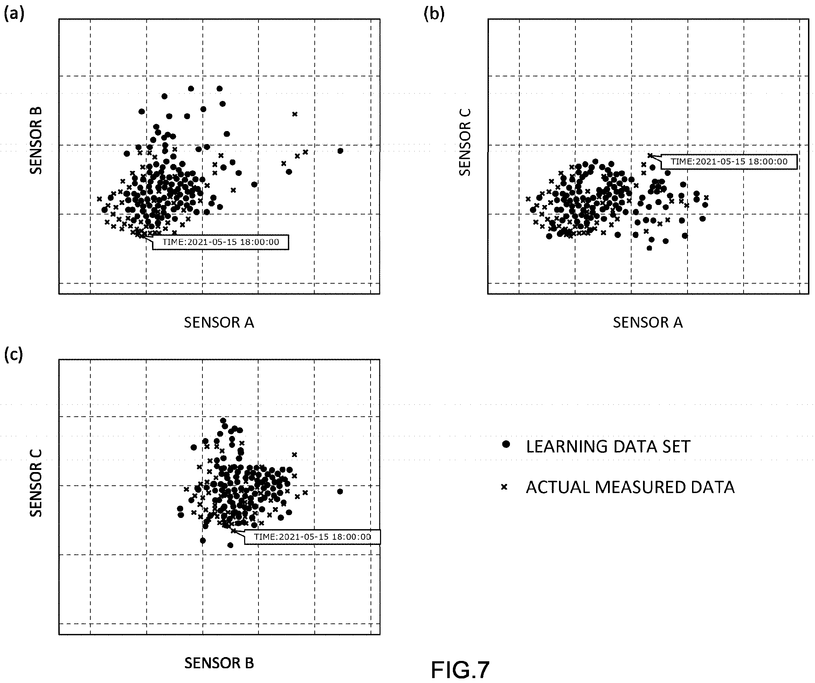

Here, in the case of a configuration including three or more sensors, as illustrated in FIG. 7, analysis graphs of all patterns selected from the sensors can be also displayed. For example, in the case of a configuration including the sensor A, the sensor B, and the sensor C, analysis graphs of the sensor A and the sensor B, analysis graphs of the sensor A and the sensor C, and analysis graphs of the sensor B and the sensor C are displayed. Here, for example, when a cursor is placed on one of the actual measured data in one analysis graph and the actual measured data is selected, actual measured data of the same time as the time of the selected actual measured data can be displayed on the other two analysis graphs in a recognizable manner. By comprehensively considering the actual measured data of three or more sensors in this manner, it is possible to analyze an event that cannot be analyzed in actual measured data of two sensors.

In addition, the time-series data is displayed on the analysis graph having two sensor axes in accordance with day of the week, month, season, or the like. This makes it possible to analyze an influence of the day of the week (for example, the factory is not operating on Saturday and Sunday), a monthly influence (for example, the busy months or off-peak months), and an influence of the seasons (for example, a busy season or an off-peak season).

### <Effects of first embodiment>

According to the measurement system 100 configured as described above, the prediction value of the second sensor 22 is estimated based on the actual measured value of the first sensor 21 using the machine learning model, the estimated prediction value of the second sensor 22 is compared with the actual measured value of the second sensor 22, and a determination is made which of the machine learning model or the measurement instrument 20 has an anomaly. Therefore, the necessity of maintenance of the measurement instrument 20 can be accurately determined. Further, in a case where the determination is made that the machine learning model has an anomaly, a detection can be made that the tendency of the sample has changed, and the machine learning model can also perform relearning.

### <Second embodiment>

Next, a second embodiment of the present invention will be described with reference to the drawings. Note that the functional units described in the first embodiment are denoted by the same reference numerals. That is, it is assumed that the measurement instrument 20 and the information processing apparatus 30 have functions described below in addition to the functions described in the first embodiment.

### <Device configuration>

The measurement system 100 according to the second embodiment of the present invention is preferably intended to the same measurement system S, for example, a flow path without inflow from upstream to downstream in the same tank or water treatment facility, but is not limited thereto.

The sensors 21 to 23 in the measurement instrument 20 measures measurement items in the same measurement system S. Among them, the first sensor 21 is preferably a sensor, such as a pH meter that is not affected by an optical system, but is not limited thereto. Further, output values output respectively from the sensors 21 to 23 are processed by the information processing apparatus 30.

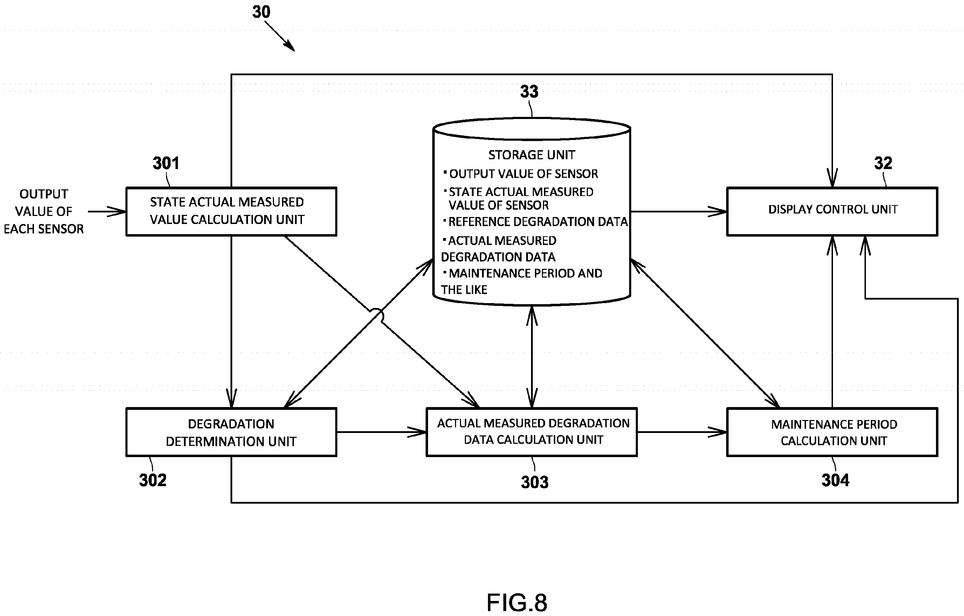

The information processing apparatus 30 delivers functionalities as the display control unit 32 and the storage unit 33 as illustrated in FIG. 8 by cooperating with the CPU 30a and peripheral devices based on a program stored in a predetermined area of the memory 30b.

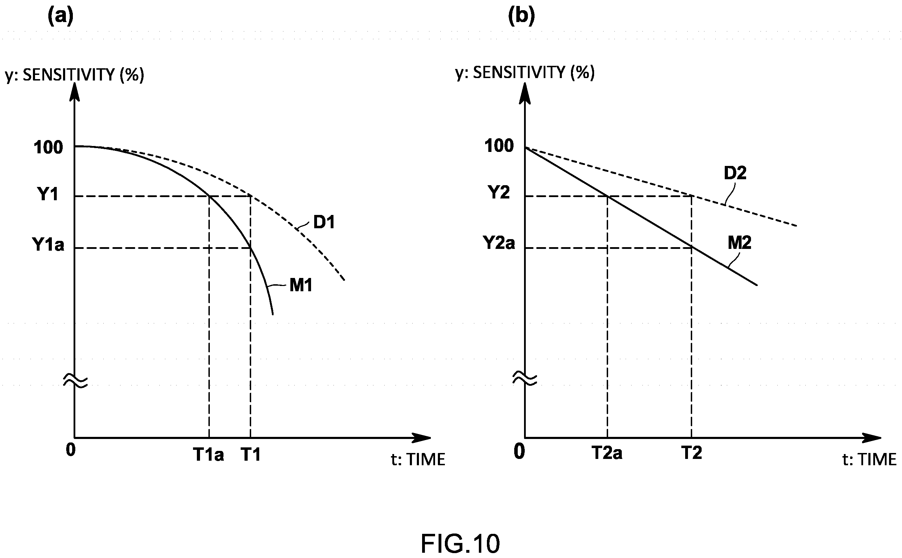

The display control unit 32 displays the output values, state values, or signals of the sensors 21 to 23 on the display. The state values mentioned here are parameters indicating the states of the sensors, and examples thereof include the sensitivities of the sensors. Specifically, as illustrated in FIG. 10, the display control unit 32 can display, for example, a graph having one axis (horizontal axis) representing time and the other axis (vertical axis) representing the sensitivity, on the display 30f. Further, the display control unit 32 can display signals generated respectively from the sensors 21 to 23 on the display 30f.

The storage unit 33 stores the output values, the state values, or the maintenance periods of the sensors 21 to 23, and stores reference degradation data D indicating a relationship between time and state prediction values that are prediction values of state values obtained by predicting the states of the sensors 21 to 23.

### <Degradation determination function>

Here, in the present embodiment, in a case where the state value in a certain sensor becomes equal to or less than a limit value (for example, 50%), a measurement failure occurs in the sensor, and the sensor needs to be replaced. Therefore, in each of the sensors 21 to 23, a reference maintenance period that is a maintenance period corresponding to the reference degradation data D is determined, and the user performs maintenance on each sensor with the reference maintenance period and checks a degradation state of each sensor. Note that in a case where a prediction is made that the state values of the sensors are equal to or less than the limit value before the reference maintenance period comes, the user replaces the sensors before maintenance.

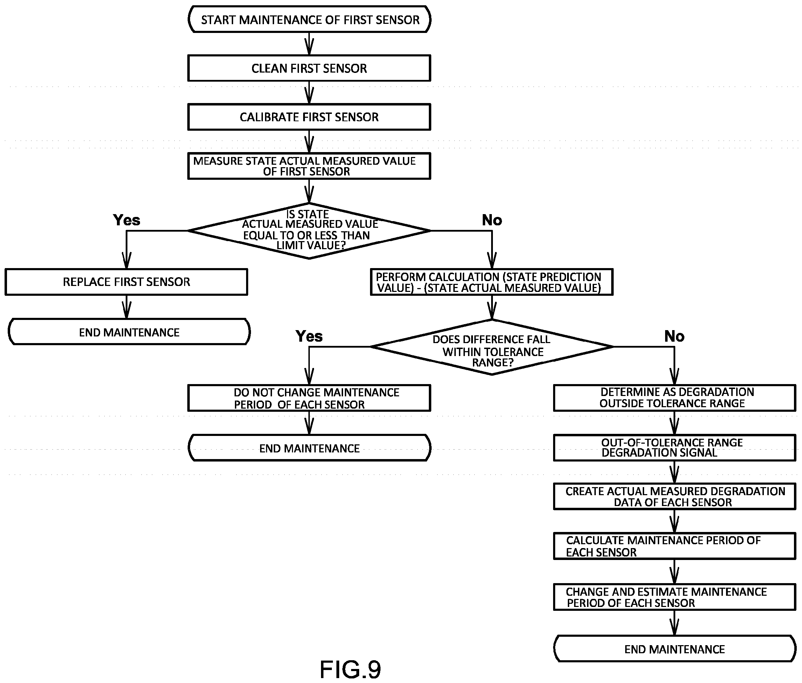

Then, as illustrated in FIG. 8, the information processing apparatus 30 further includes functions as a state actual measured value calculation unit 301, a degradation determination unit 302, an actual measured degradation data calculation unit 303, and a maintenance period calculation unit 304. Hereinafter, the functions of the state actual measured value calculation unit 301, the degradation determination unit 302, the actual measured degradation data calculation unit 303, and the maintenance period calculation unit 304 will be described with reference to a flowchart illustrated in FIG. 9. Further, for convenience of description, two of the sensors 21 to 23, that is, the first sensor 21 and the second sensor 22 will be described, but the same applies to two sensors, that is, the second sensor 22 and the third sensor 23, and two sensors, that is, the first sensor 21 and the third sensor 23.

As illustrated in FIG. 9, the user starts the maintenance of the first sensor 21 with a reference maintenance period T1. First, the user cleans the first sensor 21 and calibrates the first sensor 21. When a state value of the calibrated first sensor 21 is output, the output state value is sent to the state actual measured value calculation unit 301. Note that the second sensor 22 and the third sensor 23 are cleaned and calibrated during the maintenance of them. However, the embodiment is not limited thereto, and the second sensor 22 and the third sensor 23 may be cleaned and calibrated during the maintenance of the first sensor 21.

Next, the state actual measured value calculation unit 301 calculates a state actual measured value Y1a obtained by actually measuring the state of the first sensor 21 based on the output value output from the first sensor 21. Specifically, when the state value of the first sensor 21 during calibration is output, the state actual measured value calculation unit 301 calculates the state actual measured value Y1a corresponding to the output state value (see FIG. 10). At this time, in a case where the state actual measured value Y1a is equal to or less than the limit value, the user replaces the first sensor 21 after calibration, and the maintenance ends. On the other hand, in a case where the state value of the first sensor 21 is a value greater than the limit value, the user does not replace the first sensor 21, and the obtained state actual measured value Y1a is sent to be stored in the storage unit 33 and sent to the degradation determination unit 302.

The degradation determination unit 302 then compares the state prediction value Y1 and the state actual measured value Y1a in the first sensor 21 to determine the state of the first sensor 21. Specifically, as illustrated in FIG. 10, the degradation determination unit 302 subtracts the state actual measured value Y1a calculated by the state actual measured value calculation unit 301 from the state prediction value Y1 stored in the storage unit 33, and determines whether an obtained difference (Y1 - Y1a) falls within a tolerance range. Note that the tolerance range can be set in advance as, for example, a range of ± 10% of the state prediction value Y1.

In a case where the difference (Y1 - Y1a) falls within the tolerance range, the degradation determination unit 302 determines that the degradation of the first sensor 21 falls within the tolerance range. In this case, the user does not change the maintenance period of the first sensor 21, and estimates that the degradation of the other sensors falls within the tolerance range. Thereafter, the user performs calibration, cleaning, and the like of sensors 21 to 23, and ends the maintenance.

On the other hand, in a case where the difference (Y1 - Y1a) is out of the tolerance range, the degradation determination unit 302 determines that the degradation is out of the tolerance range, and outputs an out-of-tolerance range degradation signal indicating that the degradation of the first sensor 21 is out of the tolerance range. Here, the out-of-tolerance range degradation signal is displayed, for example, on the display 30f of the display control unit 32. Further, the out-of-tolerance range degradation signal is output to the storage unit 33 and the actual measured degradation data calculation unit 303. Then, the user is prompted to check the state value of the second sensor 22 in the same measurement system S by recognizing the out-of-tolerance range degradation signal displayed on the display 30f. Further, the state value of the second sensor 22 is output to the state actual measured value calculation unit 301, and the state actual measured value calculation unit 301 calculates a state actual measured value Y2a of the second sensor 22. The calculated state actual measured value Y2a is sent to the storage unit 33 and the actual measured degradation data calculation unit 303.

In a case where a determination is made that the degradation is out of the tolerance range, as illustrated in FIG. 10, the state actual measured value Y1a may be smaller than the state prediction value Y1, but the present invention is not limited thereto. That is, even in a case where the state actual measured value Y1a is greater than the state prediction value Y1, the degradation determination unit 302 determines that the degradation is out of the tolerance range. In this case, the state actual measured value Y1a with the reference maintenance period T1 is better than the state prediction value Y1, that is, the actual degradation rate is slower than the reference degradation data D 1.

The actual measured degradation data calculation unit 303 calculates first actual measured degradation data M 1 indicating a relationship between the state actual measured value of the first sensor 21 and time, and second actual measured degradation data M2 indicating a relationship between the state actual measured value of the second sensor 22 and time. Specifically, as illustrated in FIGS. 9 and 10, upon receiving the out-of-tolerance range degradation signal from the degradation determination unit 302, the actual measured degradation data calculation unit 303 calculates the first actual measured degradation data M1 based on the state actual measured value Y1a corresponding to the first sensor 21 and the past state actual measured value of the first sensor 21 stored in the storage unit 33. Then, the calculated first actual measured degradation data M1 is sent to the storage unit 33 and the maintenance period calculation unit 304. Note that the calculation of the second actual measured degradation data M2 in the second sensor 22 is also similar to the case of the first sensor 21.

The maintenance period calculation unit 304 calculates the maintenance periods of the first sensor 21 and the second sensor 22 based on the actual measured degradation data in the first sensor 21 and the second sensor 22. Specifically, as illustrated in FIG. 10, the maintenance period calculation unit 304 calculates an actual measured maintenance period T1a that reaches the same value as the state prediction value Y1, based on the first actual measured degradation data M1. Note that the calculation of an actual measured maintenance period T2a in the second sensor 22 is also similar to the case of the first sensor 21.

The obtained actual measured maintenance periods T1a and T2a are sent to the display control unit 32 and the storage unit 33. Then, the user changes the actual measured maintenance periods T1a and T2a displayed on the display control unit 32 to new maintenance periods, and the maintenance ends.

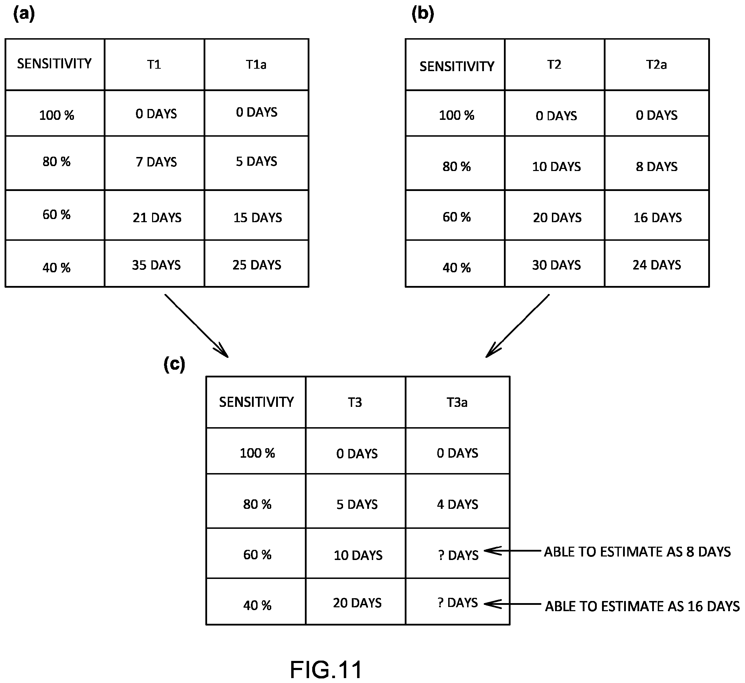

Here, as illustrated in FIG. 11, the reference maintenance periods T1 and T2 are compared with the obtained actual measured maintenance periods T1a and T2a, and the user can estimate that the degradation tendency indicating the temporal change of the state value of the same measurement system S is similar to the degradation tendency of the other sensors in the same measurement system S, based on the change rates (T1a/T1 and T2a/T2). In this case, the user can estimate, for example, an actual measured maintenance period T3a of the third sensor 23 of the same measurement system S from the change rate (T1a/T1 or T2a/T2) of the maintenance period of the first sensor 21 or the second sensor 22.

Further, the user can estimate the actual measured maintenance periods T2a and T3a of the second sensor 22 and the third sensor 23 based on the change rate (T1a/T1) of the maintenance period of the first sensor 21. Specifically, the user can estimate the actual measured maintenance periods T2a and T3a of the second sensor 22 and the third sensor 23 by multiplying the change rate (T1a/T1) of the maintenance period of the first sensor 21 by the reference maintenance periods T2 and T3 of the second sensor 22 and the third sensor 23, respectively.

On the other hand, in order to improve the accuracy of the estimation of the maintenance period, the user can estimate the actual measured maintenance period T3a of the third sensor 23 based on both the change rate (T1a/T1) of the maintenance period of the first sensor 21 and the change rate (T2a/T2) of the maintenance period of the second sensor. Specifically, the user can estimate the actual measured maintenance period T3a of the third sensor 23 by calculating an average value of the two change rates (T1a/T1 or T2a/T2) and multiplying the reference maintenance period T3 of the third sensor by the average value.

In the above embodiment, the degradation determination unit 302 calculates the difference (Y1 - Y1a) to determine the degradation state of the first sensor 21, but the user may calculate the difference (Y1 - Y1a) to determine the degradation state of the first sensor 21. In this case, when the user determines that the degradation is out of the tolerance range, the user outputs the out-of-tolerance range degradation signal to the actual measured degradation data calculation unit 303.

### <Effects of second embodiment>

According to the measurement system 100 configured as described above, the degradation determination unit 302 calculates the difference between the state actual measured value Y1a and the state prediction value Y1 of the first sensor 21, and outputs the out-of-tolerance range degradation signal in a case where the difference is out of the tolerance range. Therefore, the user can be prompted to check degradation states of other sensors in the same measurement system S. In addition, since the maintenance period calculation unit 304 calculates the actual measured maintenance periods of the sensors based on the actual measured degradation data calculated by the actual measured degradation data calculation unit 303, the maintenance period can be changed to one adaptable to the degradation states of the sensors 21 to 23 for each maintenance. In a case where the actual measured maintenance periods of the first sensor 21 and the second sensor 22 are compared with the reference maintenance period and the degradation tendencies of the sensors are similar to each other, the degradation tendencies of the other sensors can be estimated. As a result, a degradation tendency of a sensor, such as a sensor installed in a final release tank, such that that measurement cannot be stopped can also be estimated from degradation tendencies of the other sensors in the same measurement system. This makes it possible to reduce the number of times and cost of maintenance of a sensor that cannot stop measurement.

### < Other embodiments>

Note that the present invention is not limited to the above embodiments.

For example, the measurement system 100 may include a plurality of measurement instruments 20. The plurality of measurement instruments 20 is considered to be disposed at different locations such as different aeration tanks of water treatment equipment or different locations of sample piping, for example,. The information processing apparatus 30 then makes an anomaly determination on each of the measurement instruments 20 as in the above-described embodiments.

The measurement device of the present invention is not limited to the case of measuring a liquid sample to be used for water quality analysis as described above, and can also be applied to a device that measures a gas sample such as NOx, SO₂, CO, CO₂, O₂, or silica.

Some of the functions of the information processing apparatus 30 described in the above embodiments may be provided in a computer or the like different from the information processing apparatus 30. As an example, a function as a data storage unit that stores data such as output values output respectively from the sensors 21 to 23 may be provided in, for example, a cloud server or the like different from the information processing apparatus 30.

Further, the information processing apparatus 30 may not include the machine learning unit, and a machine learning model machine-learned by a computer or the like different from the information processing apparatus 30 may be transmitted to the information processing apparatus 30 via a communication line to be mounted.

In addition, the present invention is not limited to the above embodiments, and it goes without saying that various modifications can be made without departing from the gist of the present invention.

### Industrial Applicability

According to the present invention, the necessity of maintenance of a measurement instrument or the necessity of relearning of a machine learning model can be determined in a measurement system utilizing machine learning.

### Reference Signs List

- 100: measurement system
- 20: measurement instrument
- 21: first sensor
- 22: second sensor
- 30: information processing apparatus
- 31: calculation unit
- 32: display control unit
- 33: storage unit
- 34: machine learning unit
- 35: prediction value estimation unit
- 36: anomaly determination unit
- 301: state actual measured value calculation unit
- 302: degradation determination unit
- 303: actual measured degradation data calculation unit
- 304: maintenance period calculation unit

## Claims

1. A measurement system comprising:
a measurement instrument including at least a first sensor and a second sensor;
a prediction value estimation unit that estimates a prediction value of the second sensor based on an actual measured value of the first sensor using a machine learning model that has learned a relationship between the actual measured value of the first sensor and an actual measured value of the second sensor; and
an anomaly determination unit that compares the prediction value of the second sensor with the actual measured value of the second sensor to determine which of the machine learning model or the measurement instrument has an anomaly.

2. The measurement system according to claim 1, wherein the anomaly determination unit determines whether a difference or a ratio between the prediction value of the second sensor and the actual measured value of the second sensor falls within a predetermined tolerance range, and determines, in a case where the difference or the ratio is out of the tolerance range, which of the machine learning model or the measurement instrument has an anomaly.

3. The measurement system according to claim 1 or 2, wherein the anomaly determination unit determines which of the machine learning model or the measurement instrument has an anomaly using a non-parametric density estimation method.

4. The measurement system according to claim 3, wherein the anomaly determination unit determines that the machine learning model has an anomaly in a case where the anomaly determination unit determines that an anomaly occurs in a region where a data density of a learning data set with which the machine learning model is created is equal to or greater than a predetermined threshold, and determines that the machine learning model or the measurement instrument has an anomaly in a case where the anomaly determination unit determines that an anomaly occurs in a region where the data density is less than the threshold.

5. The measurement system according to any one of claims 1 to 4, further comprising a machine learning unit that creates the machine learning model using a learning data set including the actual measured value of the first sensor and the actual measured value of the second sensor.

6. The measurement system according to claim 5, wherein the machine learning unit causes the machine learning model to perform relearning in a case where the anomaly determination unit determines that the machine learning model has an anomaly.

7. The measurement system according to any one of claims 1 to 6, wherein the anomaly determination unit outputs a maintenance signal for prompting a user to perform maintenance of the measurement instrument in a case where the anomaly determination unit determines that the measurement instrument has an anomaly.

8. The measurement system according to any one of claims 1 to 7, wherein the first sensor and the second sensor each measure any one of conductivity, specific resistance, oxidation-reduction potential (ORP), chemical oxygen demand (COD), turbidity, chromaticity, temperature, pressure, or oil film of a sample, or concentration of a predetermined component contained in the sample.

9. The measurement system according to any one of claims 1 to 8, further comprising
a degradation determination unit that determines, based on reference degradation data indicating a relationship between a state prediction value obtained by predicting a state of the first sensor and time and actual measurement degradation data indicating a relationship between a state actual measured value obtained by actually measuring the state of the first sensor and time, whether a difference between the state actual measured value and the state prediction value of the first sensor falls within a tolerance range,
wherein the degradation determination unit outputs an out-of-tolerance range degradation signal indicating that the state of the first sensor is out-of-tolerance-range degradation in a case where the degradation determination unit determines that the difference is out of the tolerance range.

10. The measurement system according to claim 9, further comprising:
an actual measured degradation data calculation unit that calculates first actual measured degradation data indicating the relationship between the state actual measured value of the first sensor and time and second actual measured degradation data indicating a relationship between a state actual measured value of the second sensor and time; and
a maintenance period calculation unit that calculates a maintenance period of the first sensor and a maintenance period of the second sensor based on the first actual measured degradation data and the second actual measured degradation data.

11. The measurement system according to any one of claims 1 to 10, wherein the measurement system is used for water quality analysis.

12. A measurement system anomaly determination method for measuring a sample using at least a first sensor and a second sensor, the method comprising:
estimating a prediction value of the second sensor based on an actual measured value of the first sensor using a machine learning model that has learned a relationship between the actual measured value of the first sensor and an actual measured value of the second sensor; and
comparing the prediction value of the second sensor with the actual measured value of the second sensor to determine which of the machine learning model or the measurement instrument has an anomaly.

13. A measurement system anomaly determination program, the measurement system including at least a first sensor and a second sensor, the program causing a computer to function as:
a prediction value estimation unit that estimates a prediction value of the second sensor based on an actual measured value of the first sensor using a machine learning model that has learned a relationship between the actual measured value of the first sensor and an actual measured value of the second sensor; and
an anomaly determination unit that compares the prediction value of the second sensor with the actual measured value of the second sensor to determine which of the machine learning model or the measurement instrument has an anomaly.
